(19) Europäisches Patentamt
European Patent Office
Office européen des brevets

(11) **EP 3 020 390 A1**

(12) **EUROPÄISCHE PATENTANMELDUNG**

(43) Veröffentlichungstag:
**18.05.2016 Patentblatt 2016/20**

(51) Int Cl.:
*A61K 8/46* *(2006.01)*    *A61K 8/73* *(2006.01)*
*A61K 8/92* *(2006.01)*    *A61K 8/37* *(2006.01)*
*A61Q 5/02* *(2006.01)*

(21) Anmeldenummer: **14193046.1**

(22) Anmeldetag: **13.11.2014**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(71) Anmelder: **BASF SE**
**67056 Ludwigshafen am Rhein (DE)**

(72) Erfinder:
• **Mauer, Werner**
  **48324 Sendenhorst-Albersloh (DE)**
• **Seipel, Werner**
  **40723 Hilden (DE)**

• **Haake, Hans-Martin**
  **40699 Erkrath (DE)**
• **Cornelsen, Sybille**
  **40883 Ratingen (DE)**
• **Pellon, Guadalupe**
  **40597 Düsseldorf (DE)**
• **Glasmacher, Birgit**
  **40597 Düsseldorf (DE)**
• **Gondek, Helga**
  **40591 Düsseldorf (DE)**

(74) Vertreter: **Reinhardt, Jürgen**
  **BASF**
  **Personal Care and Nutrition GmbH**
  **Henkelstraße 67**
  **40589 Düsseldorf (DE)**

(54) **Shampoos und Spülungen mit konditionierender Wirkung**

(57)    Die vorliegende Erfindung betrifft eine Zusammensetzung, die als Shampoo oder Haarspülung geeignet ist, enthaltend mindestens ein Tensid, mindestens ein kationisches Polymer, ethoxylierte Fettsäureglyceride, ethoxyilierte Mono- und Diglycerinester, Trimethylpropan-EO/PO-Trioleat und Wasser. Weiterhin betrifft die vorliegende Erfindung ein Zwischenprodukt, das zur Herstellung der genannten Zusammensetzung geeignet ist, enthaltend ethoxylierte Fettsäureglyceride, ethoxyilierte Mono- und Diglycerinester und Trimethylpropan-EO/PO-Trioleat.

EP 3 020 390 A1

**Beschreibung**

Die vorliegende Erfindung betrifft eine Zusammensetzung, die als Shampoo oder Haarspülung geeignet ist, enthaltend mindestens ein Tensid, mindestens ein kationisches Polymer, ethoxylierte Fettsäureglyceride, ethoxyilierte Mono- und Diglycerinester, Trimethylpropan-EO/PO-Trioleat und Wasser. Weiterhin betrifft die vorliegende Erfindung ein Zwischenprodukt, das zur Herstellung der genannten Zusammensetzung geeignet ist, enthaltend ethoxylierte Fettsäureglyceride, ethoxyilierte Mono- und Diglycerinester und Trimethylpropan-EO/PO-Trioleat.

Die meisten Shampoos, die aktuell im Markt angetroffen werden, enthalten eine Kombination aus (1) einem anionischen Tensid, (2) einem Cotensid, (3) einem kationischem Polymer und (4) einem oder mehreren so genannten Emollients oder Wachsen. Dabei dienen die Emollients oder Wachse zur Konditonierung der Haare.

Als Cotensid (2) werden verschiedene Tenside verwendet, z. B. Coco-Betain, APG, Amphoacetate. Es werden also unter anderem betainische, nichtionische, und ggf auch anionische Tenside verwendet.

Als kationische Polymere (3) werden unter anderem verwendet Polyquaternium-10 (abgekürzt als PQ 10), kationisch modifizierte Cellulose, kationische Guarderivate.

Als Emollients (4) werden unter anderem native oder synthetische Öle oder Silikone verwendet.

Durch so genannte Koacervatbildung (Koazervation) (das ist die Ausfällung eines Komplexes aus Kationpolymer und Aniontensid) beim Verdünnen (Auswaschen der Haare) werden kationische Polymere und Emollients/Wachse auf dem Haar abgeschieden. Das deponierte Material reduziert dann die Reibung beim Kämmen der nassen und trockenen Haare. Gemessen wird dies als Nass- und Trockenkämmarbeit. Die als Konditioniermittel verwendeten Substanzen sind üblicherweise hydrophob. Häufig werden Silikone (Polysiloxane) als Emollient verwendet.

Unter Haarkonditionierung versteht der Fachmann die Behandlung von Haaren mit pflegenden so genannten Rinse-off-Formulierungen (also Formulierungen, die abgespült werden) oder so genannten Leave-on-Formulierungen (also Formulierungen, die auf den Haaren verbleiben, ohne abgespült zu werden), insbesondere mit pflegenden Shampoos oder Spülungen (engl. Conditioner). Diese Behandlung führt insbesondere zu einer leichteren Kämmbarkeit der Haare im nassen und trockenen Zustand, sowohl in den Längen als auch in den Spitzen (bessere Entwirrbarkeit), zu verbesserten taktilen Eigenschaften wie Glätte, Weichheit und Geschmeidigkeit sowie zu mehr Haarglanz, weniger elektrostatischer Aufladung und besserer Frisierbarkeit. Insgesamt wird somit bei der Konditionierung ein gepflegter und gesund wirkender Gesamtzustand des Haares erzielt.

Neben den oben genannten hydrophoben Substanzen zur Verbesserung der Kämmbarkeit werden hydrophile Komponenten wie z. B. Cetiol® HE (ein Kokosfettsäure-mono- und diglycerid + 7 mol Ethylenoyideinheiten (im Folgenden mit EO abgekürzt)) oder Eumulgin® CO 40 (ein hydriertes Rizinusöl mit 40 mol EO) als Solubilisatoren (das sind Lösevermittler für Öle, hydrophobe Substanzen oder auch Parfums) in Shampoos eingesetzt.

DE 10 2008 034 388 offenbart eine tensidhaltige Zusammensetzung, enthaltend neben einer oder mehrerer Ölkomponente(n) ein Gemisch aus Lösungsvermittlern, die ausgewählt sind aus der Gruppe der a) ethoxylierten Fettalkohole, b) ethoxylierten hydrierten Rizinusöle und c) ethoxylierten Mono-, Di- oder Triglycerinester, dadurch gekennzeichnet, dass das Verhältnis der Komponenten a):b):c) im Bereich von 1:(2-4):(3-4) liegt.

WO 00/64410 beschreibt die Verbesserung der Nasskämmbarkeit durch Verwendung eines Shampoos bestehend aus Tensid plus kationischem Polymer und / oder kationischem Tensid und Poly-alpha-olefin-Öl ausgehend von C6-16 Alken-Monomeren.

WO 2005/048971 beschreibt die Verwendung hydrophiler Solubilisatoren in Formulierungen zur Körperreinigung mit dem Ziel reduzierter Schädigung von Enzymen auf der Haut.

Der vorliegenden Erfindung liegt die Aufgabe zugrunde, zu den bekannten Shampoos oder Spülungen (englisch Conditioner) mit konditionierender Wirkung, die auf hydrophoben Emollients oder Wachsen basieren, eine Alternative bereit zu stellen.

Dabei sollten nach Möglichkeit Shampoos oder Spülungen mit konditionierender Wirkung bereitgestellt werden, die nicht zwingend Silikone enthalten müssen, weil silikonfreie Produkte aktuell am Markt nachgefragt werden.

Außerdem sollte nach Möglichkeit eine gute Konditionierwirkung insbesondere auch an geschädigtem Haar erzielt werden, welches durch die Schädigung hydrophil ist und damit mit hydrophoben Pflegesubstanzen eine weniger starke Wechselwirkung eingeht.

Bei der Suche nach Formulierungen für Shampoos oder Spülungen, die zu verbesserter Kämmbarkeit beitragen, wurde überraschend gefunden, dass eine Kombination von hydrophilen Solubilisatoren mit kationischen Pflegepolymeren und Tensiden auch ohne Verwendung hydrophober Substanzen eine signifikante Verbesserung der Kämmbarkeit zur Folge hatte.

**Zusammensetzung**

Die oben genannte Aufgabe wird gelöst durch eine Zusammensetzung, die als Shampoo oder Haarspülung geeignet ist, enthaltend

- mindestens ein Tensid,
- optional mindestens ein Cotensid, welches von dem mindestens einem Tensid verschieden ist,
- mindestens ein kationisches Polymer,
- ethoxylierte Fettsäureglyceride,
- ethoxyilierte Mono- und Diglycerinester,
- Trimethylpropan-EO/PO-Trioleat,
- optional Glycerin,
- optional weitere in Shampoos oder Haarspülungen übliche Inhaltsstoffe, und
- Wasser.

Diese Zusammensetzung ist ein Gegenstand der vorliegenden Erfindung.

**Tenside**

Das in der erfindungsgemäßen Zusammensetzung enthaltene mindestens eine Tensid kann ein beliebiges Tensid sein. Insbesondere kommt in Betracht ein Tensid ausgewählt aus der Gruppe bestehend aus einem Sulfat, einem ethoxilierten Sulfat, einem Sulfonat, einem Alkylpolyglycosid, einem Derivat eines Alkylpolyglycosids, einem Betain, einem Amphoacetat, einem Glutamat, einem Sulfosuccinat, einem Taurat, einem Glycinat und einem Isethionat. In einer Ausführungsform der vorliegenden Erfindung ist das mindestens eine Tensid ein anionisches Tensid, bevorzugt ein Natriumalkylethersulfat mit 12 bis 14 C-Atomen und zwei Ethylenoxieinheiten als Etherkomponente.

Das in der erfindungsgemäßen Zusammensetzung enthaltene mindestens eine Tensid ist bevorzugt in einer Menge von 10 bis 20 Gew.-% in der erfindungsgemäßen Zusammensetzung enthalten.

**Cotenside**

Das in der erfindungsgemäßen Zusammensetzung enthaltene mindestens eine Cotensid kann ein beliebiges Cotensid sein. Es ist so auszuwählen, dass es von dem mindestens eine Tensid verschieden ist. In einer Ausführungsform der vorliegenden Erfindung ist das mindestens eine Tensid ein Cocamidopropylbetain.

In einer Ausführungsform enthält die Zusammensetzung gemäß der vorliegenden Erfindung eine Kombination aus einem Natriumalkylethersulfat mit 12 bis 14 C-Atomen und zwei Ethylenoxieinheiten als Etherkomponente als Tensid und aus einem Cocamidopropylbetain als Cotensid.

Das in der erfindungsgemäßen Zusammensetzung enthaltene mindestens eine Cotensid ist bevorzugt in einer Menge von 10 bis 15 Gew.-% in der erfindungsgemäßen Zusammensetzung enthalten.

**Kationische Polymere**

Das in der erfindungsgemäßen Zusammensetzung enthaltene mindestens eine kationische Polymer kann ein beliebiges kationisches Polymer sein. In einer Ausführungsform der vorliegenden Erfindung ist das mindestens eine kationische Polymer ausgewählt aus der Gruppe bestehend aus einem kationisch modifizierten Cellulosederivat, PQ 10, PQ 67, einem kationisch modifizierte Guarderivat, Guar Hydroxypropyltrimonium Chlorid, einem kationischen Homo- oder Copolymer auf der Basis von Acrylamid, einem kationischen Homo- oder Copolymer auf der Basis von Vinylpyrrolidon, einem kationischen Homo- oder Copolymer auf der Basis von quaternisiertem Vinylimidazol und einem kationischen Homo- oder Copolymer auf der Basis von Methacrylaten.

Das in der erfindungsgemäßen Zusammensetzung enthaltene mindestens eine kationische Polymer ist bevorzugt in einer Menge von 0,1 bis 5 Gew.-% in der erfindungsgemäßen Zusammensetzung enthalten.

**Ethoxylierte Fettsäureglyceride**

Die in der erfindungsgemäßen Zusammensetzung enthaltenen ethoxyilierten Fettsäureglyceride sind ein Gemisch aus mehreren Einzelverbindungen. Sie sind erhältlich durch die Umsetzung von Fettsäureglyceriden mit Ethylenoxid (EO) im Alkalischen, z. B. in Gegenwart von KOH, bei erhöhter Temperatur, z. B. 100 bis 150 °C, wobei Umesterungen und Ethoxylierungen stattfinden, so dass das resultierende Produkt auch ethoxylierte Partialglyceride enthalten kann. In einer Ausführungsform der vorliegenden Erfindung wird ethoxiliertes, hydrierte Rizinusöle eingesetzt. In einer Ausführungsform der vorliegenden Erfindung wird ethoxiliertes, hydriertes Rizinusöl mit 40 Ethylenoxideinheiten eingesetzt.

Als Fettsäure werden im Zusammenhang mit der vorliegenden Erfindung insbesondere aliphatische Monocarbonsäuren mit unverzweigter Kohlenstoffkette, insbesondere solche mit 6 bis 30 C-Atomen bezeichnet.

Die in der erfindungsgemäßen Zusammensetzung enthaltenen ethoxyilierten Fettsäureglyceride sind bevorzugt in einer Menge von 0,06 bis 3,5 Gew.-%, bevorzugt 0,12 bis 2,1 Gew.-%, in der erfindungsgemäßen Zusammensetzung enthalten.

**Ethoxyilierte Mono- und Diglycerinester**

Die in der erfindungsgemäßen Zusammensetzung enthaltenen ethoxyilierten Mono- und Diglycerinester sind ein Gemisch aus mehreren Einzelverbindungen. Sie sind Gemische von Molekülen, die je einen Glycerinrest enthalten, und die je einen oder zwei Fettsäurereste enthalten, und die jeweils im statistischen Mittel eine bestimmte Anzahl an Ethylenoxidresten enthalten. In einer Ausführungsform der vorliegenden Erfindung werden ethoxilierte Kokosöl-Partialglyceride verwendet. In einer Ausführunsgform der vorliegenden Erfindung werden ethoxilierte Kokosöl-Partialglyceride mit 7 Ethylenoxideinheiten verwendet.

Die in der erfindungsgemäßen Zusammensetzung enthaltenen ethoxyilierten Mono- und Diglycerinester sind bevorzugt in einer Menge von 0,06 bis 2,5 Gew.-%, bevorzugt 0,12 bis 1,5 Gew.-%, in der erfindungsgemäßen Zusammensetzung enthalten.

**Trimethylpropan-EO/PO-Trioleat,**

Das in der erfindungsgemäßen Zusammensetzung enthaltene Trimethylpropan-EO/PO-Trioleat ist ein Gemisch aus mehreren Einzelverbindungen. Es ist erhältlich durch die Umsetzung von Trimethylolpropantrioleat mit Ethylenoxid und Propylenoxid unter alkalischen Bedingungen. Dabei findet, zumindest zum Teil, eine Einlagerung der Ethylenoxideinheiten (EO) und der Propylenoxideinheiten (PO) in die Estergruppen des Trimethylolpropantrioleat statt. Das Trimethylpropan-EO/PO-Trioleat wird charakterisiert durch seinen Gehalt an EO- und PO-Einheiten pro Molekül im statistischen Mittel. In einer Ausführunsgform der vorliegenden Erfindung wird Trimethylpropan-EO/PO-Trioleat, mit 120 Ethylenoxideinheiten (EO) und 10 Propylenoxideinheiten (PO) eingesetzt.

Das in der erfindungsgemäßen Zusammensetzung enthaltene Trimethylpropan-EO/PO-Trioleat ist bevorzugt in einer Menge von 0,03 bis 0,5 Gew.-%, bevorzugt 0,06 bis 0,3 Gew.-%, in der erfindungsgemäßen Zusammensetzung enthalten.

**Weitere Ausführungsformen der Zusammensetzung**

In einer Ausführungsform enthält die Zusammensetzung gemäß der vorliegenden Erfindung

- mindestens ein anionisches Tensid, bevorzugt ein Natriumalkylethersulfat mit 12 bis 14 C-Atomen und zwei Ethylenoxieinheiten als Etherkomponente,
- optional mindestens ein Cotensid, welches von dem mindestens einem Tensid verschieden ist, und welches ein Cocamidopropylbetain ist,
- mindestens ein kationisches Polymer,
- ethoxilierte, hydrierte Rizinusöle,
- ethoxilierte Kokosöl-Partialglyceride,
- Trimethylpropan-EO/PO-Trioleat,
- optional Glycerin,
- optional weitere in Shampoos oder Haarspülungen übliche Inhaltsstoffe, und
- Wasser.

In einer Ausführungsform enthält die Zusammensetzung gemäß der vorliegenden Erfindung

   10 bis 20 Gew.-% des mindestens einen Tensids,
   0 bis 15 Gew.-% des mindestens einen Cotensids,
   0,1 bis 5 Gew.-% des kationischen Polymers,
   0,06 bis 3,5 Gew.-%, bevorzugt 0,12 bis 2,1 Gew.-%, ethoxylierte Fettsäureglyceride,
   0,06 bis 2,5 Gew.-%, bevorzugt 0,12 bis 1,5 Gew.-%, ethoxyilierte Mono- und Diglycerinester,
   0,03 bis 0,5 Gew.-%, bevorzugt 0,06 bis 0,3 Gew.-%, Trimethylpropan-EO/PO-Trioleat,
   optional Glycerin,
   optional weitere in Shampoos oder Haarspülungen übliche Inhaltsstoffe enthält, und zur Ergänzung auf 100 Gew.-% Wasser.

In einer Ausführungsform enthält die Zusammensetzung gemäß der vorliegenden Erfindung weniger als 2 Gew.-%, insbesondere weniger als 1 Gew.-%, insbesondere weniger als 0,5 Gew.-% Polysiloxane.

In einer Ausführungsform enthält die Zusammensetzung gemäß der vorliegenden Erfindung weniger als 2 Gew.-%, insbesondere weniger als 1 Gew.-%, insbesondere weniger als 0,5 Gew.-% an Stoffen, die Emollients oder Wachse sind.

**Zwischenprodukt**

Ein weiterer Gegenstand der vorliegenden Erfindung ist ein Zwischenprodukt, das zur Herstellung der erfindungsgemäßen Zusammensetzung geeignet ist, enthaltend

- ethoxylierte Fettsäureglyceride,
- ethoxyilierte Mono- und Diglycerinester,
- Trimethylpropan-EO/PO-Trioleat,
- optional Wasser, und
- optional Glycerin.

Dabei haben die Begriffe ethoxylierte Fettsäureglyceride, ethoxyilierte Mono- und Diglycerinester, und Trimethylpropan-EO/PO-Trioleat dieselbe Bedeutung wie im Zusammenhang mit der erfindungsgemäßen Zusammensetzung und können insbesondere in denjenigen Ausführungsformen verwirklicht werden, wie sie in den Ausführungsformen der erfindungsgemäßen Zusammensetzung verwirklicht werden.

**Mengenanteile im Zwischenprodukt**

In einer Ausführungsform sind die Komponenten des Zwischenprodukts ((a) ethoxylierte Fettsäureglyceride, (b) ethoxyilierte Mono- und Diglycerinester, (c) Trimethylpropan-EO/PO-Trioleat) in einem Massenverhältnis von a:b:c = 2-1:1-2:0,2-0,5, insbesondere 2:1:0,3, in dem Zwischenprodukt vorhanden.

Die in dem erfindungsgemäßen Zwischenprodukt enthaltenen ethoxyilierten Fettsäureglyceride sind bevorzugt in einer Menge von 12 bis 70 Gew.-%, insbesondere 40 bis 66 Gew.-%, insbesondere 55 bis 56 Gew.-%, in dem erfindungsgemäßen Zwischenprodukt enthalten.

Die in dem erfindungsgemäßen Zwischenprodukt enthaltenen ethoxyilierten Mono- und Diglycerinester sind bevorzugt in einer Menge von 12 bis 50 Gew.-%, insbesondere 20 bis 33 Gew.-%, insbesondere 27 bis 28 Gew.-%, in dem erfindungsgemäßen Zwischenprodukt enthalten.

Das in dem erfindungsgemäßen Zwischenprodukt enthaltene Trimethylpropan-EO/PO-Trioleat ist bevorzugt in einer Menge von 6 bis 10 Gew.-%, insbesondere 8 bis 9 Gew.-%, in dem erfindungsgemäßen Zwischenprodukt enthalten.

Das in dem erfindungsgemäßen Zwischenprodukt optional enthaltene Glycerin ist bevorzugt in einer Menge von 3 bis 5 Gew.-% in dem erfindungsgemäßen Zwischenprodukt enthalten.

Das in dem erfindungsgemäßen Zwischenprodukt enthaltene Wasser ist bevorzugt in einer Menge von 6 bis 10 Gew.-%, insbesondere 4 bis 5 Gew.-%, in dem erfindungsgemäßen Zwischenprodukt enthalten.

**Weitere Ausführungsformendes Zwischenprodukts**

Eine Ausführungsform der vorliegenden Erfindung ist das Zwischenprodukt, das zur Herstellung der erfindungsgemäßen Zusammensetzung geeignet ist, enthaltend

- ethoxilierte, hydrierte Rizinusöle,
- ethoxilierte Kokosöl-Partialglyceride,
- Trimethylpropan-EO/PO-Trioleat,
- optional Wasser, und
- optional Glycerin.

Eine weitere Ausführungsform der vorliegenden Erfindung ist das Zwischenprodukt, das zur Herstellung der erfindungsgemäßen Zusammensetzung geeignet ist, enthaltend

- 12 bis 70 Gew.-%, bevorzugt 40 bis 66 Gew.-%, ethoxylierte Fettsäureglyceride, bevorzugt ethoxilierte, hydrierte Rizinusöle,
- 12 bis 50 Gew.-%, bevorzugt 20 bis 33 Gew.-%, ethoxyilierte Mono- und Diglycerinester, bevorzugt ethoxilierte Kokosöl-Partialglyceride,
- 6 bis 10 Gew.-% Trimethylpropan-EO/PO-Trioleat,

- optional Wasser, und
- optional Glycerin.

Eine weitere Ausführungsform der vorliegenden Erfindung ist das Zwischenprodukt, das zur Herstellung der erfindungsgemäßen Zusammensetzung geeignet ist, enthaltend

- 55 bis 56 Gew.-% ethoxiliertes, hydriertes Rizinusöl mit 40 Ethylenoxideinheiten,
- 27 bis 28 Gew.-% ethoxilierte Kokosöl-Partialglyceride mit 7 Ethylenoxideinheiten,
- 8 bis 9 Gew.-% Trimethylpropan-EO/PO-Trioleat, mit 120 Ethylenoxideinheiten (EO) und 10 Propylenoxideinheiten (PO),
- 4 bis 5 Gew.-% Wasser, und
- 3 bis 5 Gew.-% Glycerin.

Üblicherweise wird das Zwischenprodukt der erfindungsgemäßen Zusammensetzung in einer Menge von 0,5 bis 5 Gew.-%, bevorzugt 1 bis 3 Gew.-% zugegeben.

**Weitere Gegenstände der Erfindung**

Ein weiterer Gegenstand der vorliegenden Erfindung ist die Verwendung des erfindungsgemäßen Zwischenproduktes zur Herstellung der erfindungsgemäßen Zusammensetzung.

Ein weiterer Gegenstand der vorliegenden Erfindung ist ein Verfahren zur Herstellung der erfindungsgemäßen Zusammensetzung umfassend das Bereitstellen des erfindungsgemäßen Zwischenproduktes und das in Kontakt Bringen des Zwischenproduktes mit den übrigen Bestandteilen der Zusammensetzung.

Ein weiterer Gegenstand der vorliegenden Erfindung ist die Verwendung der erfindungsgemäßen Zusammensetzung zur Konditionierung von Haaren, bevorzugt von menschlichen Haaren.

Ein weiterer Gegenstand der vorliegenden Erfindung ist die Verwendung der erfindungsgemäßen Zusammensetzung zur Verbesserung der Kämmbarkeit, bevorzugt zur Verbesserung der Nasskämmbarkeit, von Haaren, bevorzugt von menschlichen Haaren. Eine besondere Ausführungsform dieser Verwendung ist gegeben, wenn die Haare geschädigt und hydrophil sind.

**Beispiele**

Wenn nichts anderes angegeben ist, bedeutet % im folgenden Gewichts-%.

Wenn nichts anderes angegeben ist, bedeutet RT oder Raumtemperatur im Folgenden 20° C.

EO bedeutet Ethylenoxy-Einheiten.

Beschreibung der in den folgenden Beispielen verwendeten Markenprodukte gemäß INCI:

| | |
|---|---|
| Texapon® N 70 | Sodium Laureth Sulfate + 2 EO |
| Dehyton® PK 45 | Cocamidopropyl Betaine |
| Polymer JR 400 | Polyquaternium-10 |
| Arlypon® TT liquid | Trimethylolpropan + 120 EO/10 PO - Random - tri - Oleat |
| Cetiol® HE | Coco, Mono- and Diglyceride + 7 mol EO |
| Eumulgin® CO 40 | hydrogenated Castor Oil + 40 mol EO |
| Dehyquart® CC7 BZ | Polyquaternium-7 |
| Dehyquart® Guar N | Guar Hydroxypropyltrimonium Chloride |
| Dehyquart® Guar TC | Guar Hydroxypropyltrimonium Chloride |
| Dehyquart® Guar HP | Guar Hydroxypropyltrimonium Chloride |
| Euperlan® PK 710 Benz | Glycol Distearate and Sodium Laureth Sulfate and Cocamide MEA |

Es wurden Schampoos gemäß der in der folgenden Tabelle angegebenen Zusammensetzung in Gew.-% hergestellt.

| Beispiele 12-278- | 04 | 05 | 06 | 07 | 08 |
|---|---|---|---|---|---|
| Texapon® N 70 | 15,70 | 15,70 | 15,70 | 15,70 | 15,70 |
| Dehyton® PK 45 | 7,40 | 7,40 | 7,40 | 7,40 | 7,40 |

(fortgesetzt)

| Beispiele 12-278- | 04 | 05 | 06 | 07 | 08 |
|---|---|---|---|---|---|
| Polymer® JR 400 | 0,50 | 0,50 | 0,50 | 0,50 | 0,50 |
| Arlypon® TT liquid | - | 0,30 | 0,30 | 0,30 | - |
| Cetiol® HE | - | - | 1,0 | 1,0 | 1,0 |
| Eumulgin® CO 40 | - | - | - | 2,0 | 2,0 |
| Parfum Cotton Touch | 0,20 | 0,20 | 0,20 | 0,20 | 0,20 |
| Na-Benzoat | 0,50 | 0,50 | 0,50 | 0,50 | 0,50 |
| Zitronensäure 50 % in Wasser | 0,90 | 0,87 | 0,97 | 0,77 | 0,90 |
| NaCl | 0,70 | 0,70 | 0,70 | 0,70 | 0,70 |
| Wasser | 74,10 | 73,83 | 72,73 | 70,93 | 71,10 |
| pH-Wert | 4,73 | 4,70 | 4,74 | 4,70 | 4,87 |
| Viskosität bei RT [mPas] * | 5000 | 25000 | 36000 | 4400 | 600 |
| NK % ** | 87+-10 | 72+-4 | 60+-9 | 39+-4 | 45+-5 |

* Die Viskosität der erhaltenen Schampoos wurde mittels eines Brookfield Viskosimerters Typ RTV DV-II bei 20 °C gemessen.

** Die Nasskämmarbeit in % des Referenzwertes (NK %) wurde gemäß der folgenden Methode bestimmt.

Die Messungen wurden jeweils an 10 Haarsträhnen in einem automatisierten System zur Bestimmung der Nasskämmarbeit durchgeführt.

Die Vorbehandlung der Haarsträhnen (12cm/1g) der Firma IHIP wurden in einem automatisierten Haarbehandlungssystem in folgenden Schritten durchgeführt:

- 30 min Reinigung mit 6 % Natriumlaurylethersulfat, pH 6.5, danach intensives Spülen der Haare,
- 20 min Bleiche mit einer Lösung von 5 % Wasserstoffperoxid, pH 9.4 (mit Ammoniumhydroxidlösung eingestellt), danach intensives Spülen der Haare,
- 30 min Trocknung in einem Luftstrom bei 68° C.

Direkt vor der Nullmessung wurden die Haare für 30 Minuten in Wasser gequollen und anschließend mit einer automatischen Nassauskämmapparatur für 1 Minute ausgespült. Im automatisierten System zur Bestimmung der Nass- und Trockenkämmarbeit wurden die Kämmkräfte während 20 Kämmungen bestimmt und die Kämmarbeit durch Integration der gemessenen Kraft-Weg-Kurven errechnet. Nach der Nullmessung wurden die Haare sofort mit der Formulierung behandelt (0,25g/g Haar). Nach 5 Minuten Einwirkzeit wurde mit der automatischen Nassauskämmapparatur unter Standardbedingungen (38°C, 1l/Minute) gespült. Die Behandlung und das folgende Ausspülen wurde ein zweites Mal wiederholt. Dann erfolgte die Vergleichsmessung (zur Nullmessung). Die Messungen wurden mit der feinen Kammseite der Naturkautschukkämme durchgeführt. Berechnet wurde die Restkämmarbeit pro Strähne wie folgt:

$$\text{Restkämmarbeit} = \text{Kämmarbeit vor Produktbehandlung/Kämmarbeit nach Produktbehandlung}$$

Die Restkämmbarkeit ist der Wert "NK %".

Anschließend wurde über die Quotienten aller 10 Strähnen der Mittelwert und die Standardabweichung bestimmt.

Aus den oben genannten Beispielen konnten die folgenden Schlüsse gezogen werden. Die Rezeptur -08 zeigte eine deutlich bessere NK im Vergleich zur Rezeptur -04. Eine weitere Verbesserung der Nasskämmbarkeit konnte durch die Verwendung des Verdickers Arlypon® TT liquid erzielt werden (Rezeptur -07). Gleichzeitig konnte der viskositätserniedrigende Effekt der Solubilisatoren aufgehoben werden.

Die als vorteilhaft aufgefundene Kombination aus den hydrophilen Solubilisatoren plus Verdicker wurde zu einem Produkt formuliert, dem "Hydrophilen Conditioning Compound". Zum Klarstellen wurde dem Produkt eine geringe Menge Wasser zugegeben Dieser Compound setzte sich folgendermaßen zusammen:

Zusammensetzung des **"Hydrophilen Conditioning Compound"** (INCI-Nomenklatur):

55,6 % Eumulgin® CO 40: hydrogenated Castor Oil + 40 mol EO
27,8 % Cetiol® HE: Coco, Mono- and Diglyceride + 7 mol EO
8,3 % Arlypon® TT liquid: Trimethylolpropan + 120 EO/10 PO - Random - tri - Oleat
8,3 % Wasser entmineralisiert

Der konditionierende Effekt (NK-Verbesserung) des hydrophilen Compounds konnte auch bei Verwendung weiterer Conditionierpolymere in klaren und perglänzenden Systemen nachgewiesen werden. Dies zeigen die Beispiele der folgenden Tabelle (Zusammensetzung in Gew.-%).

| Beispiele 14-017- | 01 | 02 | 05 | 21 | 22 | 23 | 24 |
|---|---|---|---|---|---|---|---|
| **Texapon® N 70** | 14,30 | 14,30 | 14,30 | 14,30 | 14,30 | 14,30 | 14,30 |
| **Dehyton® PK 45** | 5,40 | 5,40 | 5,40 | 5,40 | 5,40 | 5,40 | 5,40 |
| **Salcare® SC 60** | 0,10 | - | - | 0,10 | - | - | - |
| **Polymer JR 400** | - | 0,20 | - | - | 0,20 | - | - |
| **Dehyquart® Guar HP** | - | - | - | - | - | 0,20 | - |
| **Dehyquart® Guar N** | - | - | - | - | - | - | 0,20 |
| **Dehyquart® Guar TC** | - | - | 0,20 | - | - | - | - |
| **Euperlan® PK 710** | - | - | - | 3,0 | 3,0 | 3,0 | 3,0 |
| **Hydrophiler Compound** | 2,0 | 2,0 | 2,0 | 2,0 | 2,0 | 2,0 | 2,0 |
| **Parfum Cotton Touch** | 0,50 | 0,50 | 0,50 | 0,50 | 0,50 | 0,50 | 0,50 |
| **Na-Benzoat** | 0,50 | 0,50 | 0,50 | 0,50 | 0,50 | 0,50 | 0,50 |
| **Zitronensäure 50 % in Wasser** | 0,72 | 0,70 | 0,78 | 0,84 | 0,77 | 0,82 | 0,75 |
| **NaCl** | 2,21 | 2,62 | 2,37 | 1,72 | 1,67 | 1,38 | 1,37 |
| **Wasser** | 74,27 | 73,78 | 73,95 | 71,64 | 71,66 | 71,90 | 71,98 |
| **Viskosität (mPas)** | 5000 | 6100 | 6300 | 6040 | 5680 | 5800 | 5400 |
| **NK (%)** | 58 | 63 | 64 | 51 | 64 | 47 | 66 |
| **Standardabweichung** | 6 | 3 | 5 | 3 | 4 | 3 | 4 |
| **TK (%)** | 105 | | | | | 62 | |

Die Rezeptur -23 zeigte auch eine erhebliche Verbesserung der Nasskämmbarkeit bei besonders stark geschädigtem Haar (3 fach bebleicht). Die NK der Formulierung -23 betrug 52 % (+- 4 %), dagegen die Placeboformulierung ohne den hydophilen Compound 101% (+- 12 %).
TK bedeutet Trockenkämmbarbeit. Der Vergleich zeigt einen deutlich besseren Wert von Beispiel 23 verglichen mit Beispiel 1.
Auch **Variationen in der Zusammensetzung des Hydrophilen Conditioning Compounds** zeigten ähnliche Ergebnisse bei der Verbesserung des Kämmbarkeit. Dies zeigen die Beispiele der folgenden Tabelle (Zusammensetzung in Gew.-%).

| Beispiele 14-017- | 29 | 30 | 31 | 32 |
|---|---|---|---|---|
| **Texapon® N 70** | 14,30 | 14,30 | 14,30 | 14,30 |
| **Dehyton® PK 45** | 5,40 | 5,40 | 5,40 | 5,40 |
| **Polymer JR 400** | 0,20 | 0,20 | 0,20 | 0,20 |

(fortgesetzt)

| Beispiele 14-017- | 29 | 30 | 31 | 32 |
|---|---|---|---|---|
| Dehyquart® Guar TC | - | - | - | - |
| Hydrophiler Compound (Alternative 1) | 2,00 | - | - | - |
| Hydrophiler Compound (Alternative 2) | - | 2,00 | - | - |
| Hydrophiler Compound (Alternative 3) | - | - | 2,00 | - |
| Hydrophiler Compound (Alternative 4) | - | - | - | 2,00 |
| Parfum Cotton Touch | 0,50 | 0,50 | 0,50 | 0,50 |
| Na-Benzoat | 0,50 | 0,50 | 0,50 | 0,50 |
| Zitronensäure 50 % in Wasser | 0,73 | 0,73 | 1,08 | 0,65 |
| NaCl | 2,50 | 2,23 | 1,56 | 1,18 |
| Wasser | 73,87 | 74,14 | 74,46 | 75,27 |
| NK (%) | 62 | 60 | 61 | 57 |
| Standardabweichung | +-4,3 | +-4,3 | +-5,3 | +-3,3 |

Hydrophiler Compound Alternative 1:
  8,3 % Arlypon® TT liquid
  55,6 % Eumulgin® HRE 40
  27,8 % Cetiol® 767
  8,3 % Wasser entmineralisiert

Hydrophiler Compound Alternative 2:
  8,3 % Arlypon® TT liquid
  55,6 % Eumulgin® HRE 40
  27,8 % Cremophor® WO7
  8,3 % Wasser entmineralisiert

Hydrophiler Compound Alternative 3:
  8,3 % Arlypon® TT liquid
  55,6 % Eumulgin® HRE 60
  27,8 % Cetiol® HE
  8,3 % Wasser entmineralisiert

Hydrophiler Compound Alternative 4:
  8,3% Arlypon® TT liquid
  27,8% Eumulgin® HRE 40
  55,6% Cetiol® HE
  8,3% Wasser entmineralisiert

In Versuchen zur **mikrobiellen Stabilität des Zwischenprodukts (hydrophiler Conditioning Compound)** erwies sich ein Wassergehalt von 8,3 % als hoch in Bezug auf die Wasseraktivität. Daher wurde in einer Variation ein Teil des Wassers durch Glycerin ersetzt. Dabei wurden die 8,3 % Wasser auf 4,3 % Wasser reduziert und 4 % Glycerin zugegeben, womit die Wasseraktivität in einen mikrobiell unbedenklichen Bereich abgesenkt wurde.

Eine Veränderung der vorteilhaften Eigenschaften des Zwischenprodukts im Hinblick auf seine Beitrag zur Konditionierwirkung daraus hergestellter Zusammensetzungen durch den partiellen Austausch des Wassers gegen Glycerin ist nicht zu erwarten.

Eine mögliche Zusammensetzung des Compounds lautet daher:

Eumulgin® CO 40 (55,6 %): hydrogenated Castor Oil + 40 mol EO
Cetiol® HE (27,8 %): Coco, Mono- and Diglyceride + 7 mol EO
Arlypon® TT liquid (8,3 %): Trimethylolpropan + 120 EO/10 PO - Random - tri-Oleat
Water entmineralisiert (4,3 %)
Glycerin (4,0 %)

**Patentansprüche**

1. Eine Zusammensetzung, die als Shampoo oder Haarspülung geeignet ist, enthaltend

- mindestens ein Tensid,
- optional mindestens ein Cotensid, welches von dem mindestens einem Tensid verschieden ist,
- mindestens ein kationisches Polymer,
- ethoxylierte Fettsäureglyceride,
- ethoxyilierte Mono- und Diglycerinester,
- Trimethylpropan-EO/PO-Trioleat,
- optional Glycerin,
- optional weitere in Shampoos oder Haarspülungen übliche Inhaltsstoffe, und
- Wasser.

2. Die Zusammensetzung nach Anspruch 1, wobei diese Zusammensetzung 10 bis 20 Gew.-% des mindestens einen Tensids enthält,

0 bis 15 Gew.-% des mindestens einen Cotensids enthält,
0,1 bis 5 Gew.-% des kationischen Polymers enthält,
0,06 bis 3,5 Gew.-% ethoxylierte Fettsäureglyceride enthält,
0,06 bis 2,5 Gew.-% ethoxylierte Mono- und Diglycerinester enthält,
0,03 bis 0,5 Gew.-% Trimethylpropan-EO/PO-Trioleat enthält,
optional Glycerin enthält,
optional weitere in Shampoos oder Haarspülungen übliche Inhaltsstoffe enthält, und
zur Ergänzung auf 100 Gew.-% Wasser enthält.

3. Die Zusammensetzung nach Anspruch 1 oder 2, wobei das kationische Polymer ausgewählt wird aus der Gruppe bestehend aus einem kationisch modifizierten Cellulosederivat, PQ 10, PQ 67, einem kationisch modifizierte Guarderivat, Guar Hydroxypropyltrimonium Chlorid, einem kationischen Homo- oder Copolymer auf der Basis von Acrylamid, einem kationischen Homo- oder Copolymer auf der Basis von Vinylpyrrolidon, einem kationischen Homo- oder Copolymer auf der Basis von quaternisiertem Vinylimidazol und einem kationischen Homo- oder Copolymer auf der Basis von Methacrylaten.

4. Die Zusammensetzung nach einem der Ansprüche 1 bis 3, wobei das mindestens eine Tensid ausgewählt wird aus der Gruppe bestehend aus einem Sulfat, einem ethoxilierten Sulfat, einem Sulfonat, einem Alkylpolyglycosid, einem Derivat eines Alkylpolyglycosids, einem Betain, einem Amphoacetat, einem Glutamat, einem Sulfosuccinat, einem Taurat, einem Glycinat und einem Isethionat.

5. Die Zusammensetzung nach einem der der Ansprüche 1 bis 4, wobei die Zusammensetzung weniger als 2 Gew.-% Polysiloxane enthält.

6. Die Zusammensetzung nach einem der der Ansprüche 1 bis 5, wobei die Zusammensetzung weniger als 2 Gew.-% an Stoffen enthält, die Emollients oder Wachse sind.

7. Ein Zwischenprodukt, das zur Herstellung der Zusammensetzung nach einem der der Ansprüche 1 bis 6 geeignet ist, enthaltend

- ethoxylierte Fettsäureglyceride,
- ethoxyilierte Mono- und Diglycerinester,
- Trimethylpropan-EO/PO-Trioleat,
- optional Wasser, und
- optional Glycerin.

8. Das Zwischenprodukt nach Anspruch 7 enthaltend

- 12 bis 70 Gew.-% ethoxylierte Fettsäureglyceride,
- 12 bis 50 Gew.-% ethoxyilierte Mono- und Diglycerinester,
- 6 bis 10 Gew.-% Trimethylpropan-EO/PO-Trioleat,
- optional Wasser, und
- optional Glycerin.

9. Das Zwischenprodukt nach Anspruch 7 enthaltend

- 55 bis 56 Gew.-% ethoxiliertes, hydriertes Rizinusöl mit 40 Ethylenoxideinheiten,
- 27 bis 28 Gew.-% ethoxilierte Kokosöl-Partialglyceride mit 7 Ethylenoxideinheiten,
- 8 bis 9 Gew.-% Trimethylpropan-EO/PO-Trioleat mit 120 Ethylenoxideinheiten (EO) und 10 Propylenoxideinheiten (PO),
- 4 bis 5 Gew.-% Wasser, und
- 3 bis 5 Gew.-% Glycerin.

10. Die Verwendung des Zwischenproduktes nach einem der Ansprüche 7 bis 9 zur Herstellung der Zusammensetzung nach einem der Ansprüche 1 bis 6.

11. Ein Verfahren zur Herstellung der Zusammensetzung nach einem der Ansprüche 1 bis 6 umfassend

- das Bereitstellen des Zwischenproduktes nach einem der Ansprüche 7 bis 9 und
- das in Kontakt Bringen des Zwischenproduktes mit den übrigen Bestandteilen der Zusammensetzung.

12. Die Verwendung der Zusammensetzung nach einem der der Ansprüche 1 bis 6 zur Konditionierung von Haaren, bevorzugt von menschlichen Haaren.

13. Die Verwendung der Zusammensetzung nach einem der der Ansprüche 1 bis 6 zur Verbesserung der Kämmbarkeit, bevorzugt zur Verbesserung der Nasskämmbarkeit, von Haaren, bevorzugt von menschlichen Haaren.

14. Die Verwendung nach Anspruch 12 oder 13, wobei die Haare geschädigt und hydrophil sind.

Europäisches Patentamt

European Patent Office

Office européen des brevets

**EUROPÄISCHER RECHERCHENBERICHT**

Nummer der Anmeldung

EP 14 19 3046

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DATABASE GNPD [Online] MINTEL; 30. Juli 2010 (2010-07-30), L'Occitane: "L'Occitane en Provence, Aromachologie 5 Essential Oils", XP002738731, Database accession no. 1363498 * das ganze Dokument * ----- | 1-5,7, 12-14 | INV. A61K8/46 A61K8/73 A61K8/92 A61K8/37 A61Q5/02 |
| Y | WO 2014/177292 A1 (HENKEL AG & CO KGAA [DE]) 6. November 2014 (2014-11-06) * Ansprüche 1-10 * * Beispiele 1,2 * ----- | 1-14 | |
| Y | DE 10 2008 034388 A1 (HENKEL AG & CO KGAA [DE]) 28. Januar 2010 (2010-01-28) * Ansprüche 1,12,14 * * Absatz [0222] * * Absätze [0006], [0009] * ----- | 1-14 | |
| Y | WO 2008/155073 A2 (COGNIS IP MAN GMBH [DE]; HLOUCHA MATTHIAS [DE]; HAAKE HANS-MARTIN [DE]) 24. Dezember 2008 (2008-12-24) * Ansprüche 1-5 * * Seite 1, Absatz 5 * * Tabellen 3,4 * ----- | 1-14 | RECHERCHIERTE SACHGEBIETE (IPC) A61K A61Q |
| Y | WO 2013/007473 A2 (COGNIS IP MAN GMBH [DE]; HLOUCHA MATTHIAS [DE]; KUESTERS ESTHER [DE];) 17. Januar 2013 (2013-01-17) * Tabelle 2 * * Ansprüche 11,12 * * Seite 23, Absatz 3 - Seite 24, Absatz 1 * ----- | 1-14 | |

Der vorliegende Recherchenbericht wurde für alle Patentansprüche erstellt

| Recherchenort | Abschlußdatum der Recherche | Prüfer |
|---|---|---|
| München | 21. April 2015 | Wörth, Christian |

EPO FORM 1503 03.82 (P04C03)

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

......................................................................

& : Mitglied der gleichen Patentfamilie, übereinstimmendes Dokument

**ANHANG ZUM EUROPÄISCHEN RECHERCHENBERICHT
ÜBER DIE EUROPÄISCHE PATENTANMELDUNG NR.**

EP 14 19 3046

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-04-2015

| Im Recherchenbericht angeführtes Patentdokument | Datum der Veröffentlichung | Mitglied(er) der Patentfamilie | Datum der Veröffentlichung |
|---|---|---|---|
| WO 2014177292 A1 | 06-11-2014 | DE 102013208055 A1<br>WO 2014177292 A1 | 06-11-2014<br>06-11-2014 |
| DE 102008034388 A1 | 28-01-2010 | DE 102008034388 A1<br>EP 2328544 A2<br>US 2011124542 A1<br>WO 2010009978 A2 | 28-01-2010<br>08-06-2011<br>26-05-2011<br>28-01-2010 |
| WO 2008155073 A2 | 24-12-2008 | CN 101808613 A<br>EP 2164449 A2<br>JP 5520820 B2<br>JP 2010530392 A<br>US 2011142778 A1<br>US 2013149272 A1<br>WO 2008155073 A2 | 18-08-2010<br>24-03-2010<br>11-06-2014<br>09-09-2010<br>16-06-2011<br>13-06-2013<br>24-12-2008 |
| WO 2013007473 A2 | 17-01-2013 | CN 103648468 A<br>EP 2729112 A2<br>JP 2014520770 A<br>KR 20140044894 A<br>US 2014219946 A1<br>WO 2013007473 A2 | 19-03-2014<br>14-05-2014<br>25-08-2014<br>15-04-2014<br>07-08-2014<br>17-01-2013 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008034388
- WO 0064410 A

- WO 2005048971 A